(19) Europäisches Patentamt

European Patent Office

Office européen des brevets

(11) **EP 0 600 347 B1**

(12) **EUROPÄISCHE PATENTSCHRIFT**

(45) Veröffentlichungstag und Bekanntmachung des Hinweises auf die Patenterteilung:
**28.02.2001 Patentblatt 2001/09**

(51) Int Cl.[7]: **A61K 47/48**, A61K 33/26, A61K 31/295

(21) Anmeldenummer: **93118828.8**

(22) Anmeldetag: **23.11.1993**

(54) **Verfahren zur selektiven Elimination von anorganischem Phosphat aus Flüssigkeiten mittels mit polynuklearen Metalloxidhydroxiden modifizierten Adsorptionsmaterialien**

Process for the selective elemination of anorganic phosphate from liquids by adsorptionmaterials modified with polymuclear metal oxide hydroxides

Procédé pour l'élimination sélective des phosphates de liquides à l'aide de matériaux d'adsorption modifiés par des oxyde-hydroxides de métaux polyucléaires

(84) Benannte Vertragsstaaten:
**AT BE CH DE ES FR GB IT LI LU NL SE**

(30) Priorität: **24.11.1992 DE 4239442**

(43) Veröffentlichungstag der Anmeldung:
**08.06.1994 Patentblatt 1994/23**

(73) Patentinhaber: **SeBo GmbH**
**64711 Erbach (DE)**

(72) Erfinder:
• **Boos, Karl-Siegfried, Prof. Dr.**
  **D-82131 Gauting (DE)**
• **Seidel, Dietrich, Prof. Dr.**
  **D-82340 Feldafing (DE)**
• **Spengler, Klaus**
  **D-34305 Niedenstein-Kirchberg (DE)**
• **Henke,Gudrun**
  **D-34212 Melsungen (DE)**
• **Rauh, Andreas, Dr.**
  **D-34119 Kassel (DE)**

(74) Vertreter: **Huber, Bernhard, Dipl.-Chem. et al**
**Weickmann & Weickmann**
**Patentanwälte**
**Postfach 86 08 20**
**81635 München (DE)**

(56) Entgegenhaltungen:
**US-A- 4 927 756**

• **J. CHROMATOGR., NL, 1989, VOL. 481, PAGE(S) 175-186, HJERTEN S. ET AL 'High-performance adsorption chromatography of proteins on deformed non-porous agarose beads coated with insoluble metal compounds. I. Coating: Ferric oxyhydroxide with stoichiometrically bound phosphate'**

- J. CHROMATOGR., NL, 1989, VOL. 481, PAGE(S) 187-199, HJERTEN S. ET AL 'High-performance adsorption chromatography of proteins on deformed non-porous agarose beads coated with insoluble metal compounds. II. Coating: Aluminium and zirconium (hydr)oxide with stoichiometrically bound phosphate'
- ARZNEIMITTEL FORSCHUNG. DRUG RESEARCH Bd. 17, Nr. 8 , August 1967 , AULENDORF DE A. MÜLLER 'Makromolekulare Eisen(III)-Hydroxid-Komplexe'
- S. BUDAVARI ET AL. (ED) 'The Merck Index, 11th Edition' 1989 , MERCK & CO , RAHWAY, US * Seite 632, Absatz 3975 *
- ACTA PHARMACOL. TOXICOL., DK, 1986, VOL. 59, SUPPL. 7, PAGE(S) 475-477, EYBL V. ET AL 'Interaction of chelating agents, ferric dextran and zinc with indium in mice'
- INT. J. NUCL. MED. BIOL., 1982, VOL. 9, NO. 4, PAGE(S) 277-82 CHOY, D. ET AL 'The effect of iron-dextran on the biodistribution of technetium pyrophosphate'
- AM. J. CLIN. NUTR., US, 1992, VOL. 56, NO. 3, PAGE(S) 533-536, HELDENBERG D. ET AL 'Effect of iron on serum 25-hydroxyvitamin D and 24,25-dihydroxyvitamin D concentrations'
- J PHARM SCI, VOL. 73, NO.6, PAGE(S) 774-780, 1984. BOUDINOT.F.D. ET AL 'FLUID SHIFTS AND OTHER FACTORS AFFECTING PLASMA PROTEIN BINDING OF PREDNISOLONE BY EQUILIBRIUM DIALYSIS.'
- AM. J. NURS., US, 1987, VOL. 87, NO. 7, PAGE(S) 933-942, NOVA G. 'Dialyzable drugs'
- DATABASE WPI Section Ch, Week 8522, Derwent Publications Ltd., London, GB; Class D16, AN 85-130825 & JP-A-60 066 978 (YAKULT HONSHA KK) 17. April 1985

## Beschreibung

Die Erfindung betrifft die selektive Elimination von anorganischem Phosphat aus Flüssigkeiten, insbesondere aus proteinhaltigen Körperflüssigkeiten wie Vollblut, Plasma, Darminhaltflüssigkeit sowie Dialysierflüssigkeit und die Herstellung eines hierzu geeigneten Arzneimittels.

Chronisch niereninsuffiziente Patienten können bekannterweise mit Hilfe der Dialyse (Hämo- oder Peritonealdialyse; künstliche Niere) über Jahre erfolgreich behandelt und somit langfristig am Leben erhalten werden. Der Entzug harnpflichtiger Stoffe durch die künstliche Niere erfolgt über eine semipermeable Membran. Dabei werden die Qualität und Quantität des Stofftransports von einer Reihe von Faktoren, wie der Oberfläche, der Struktur und der Dicke der Dialysemembran, den Flußraten der Spüllösung (Dialysierflüssigkeit) und des Blutes, der Ultrafiltrationsrate, der Dauer der Dialysebehandlung, dem Konzentrationsunterschied der dialysierbaren Substanzen zwischen Blut und Spüllösung sowie der Molekülgröße und -form dialysierbarer Substanzen bestimmt.

Im Verlaufe einer Dauerhämodialyse treten jedoch oftmals Komplikationen auf, die für diese Patientengruppe charakteristisch sind. So gehört die renale Osteopathie zu den schwerwiegenden Langzeitkomplikationen. Der Dialysepatient wird durch diese Krankheit nicht nur in seinem Allgemeinbefinden erheblich beeinträchtigt, sondern darüber hinaus auch von Invalidität bedroht. Eine Komponente dieses Krankheitsbildes ist der sekundäre Hyperparathyreoidismus, der mit einer urämischen Hyperphosphatämie einhergeht. Die chronische Akkumulation von Phosphat bei Dialysepatienten führt zu stark erhöhten Serumkonzentrationen an anorganischem Phosphor (größer 6 mg/dl) und beruht auf einer durch die Dialysemembran reduzierten Phosphat-Clearance.

Ein Ziel der prophylaktischen und therapeutischen Maßnahmen bei Patienten mit renaler Osteodystrophie ist daher primär die Senkung des Serumphosphatspiegels unter einen Schwellenwert von 1,8 mmol/l (5,6 mg Phospor/dl). Eine diätetische Restriktion der Phosphatzufuhr und damit eine wirkungsvolle Senkung des Serumphosphatspiegels ist in der Langzeittherapie begrenzt, wenn nicht unmöglich, da die Gefahr einer nicht ausreichenden Proteinzufuhr und somit einer Malnutrition besteht. So kann beispielsweise bei einer durchschnittlichen diätetischen Phosphatzufuhr von 3,8 bis 4,7 g/Tag durch die Dialyse (Hämo- oder Peritonealdialyse) nur etwa 1 g Phosphat pro Tag eliminiert werden. Die Patienten weisen somit trotz Restriktion eine unerwünschte, positive Phosphatbilanz auf (Hercz, G. et al., Kidney Int.Suppl. 22 (1987), 215-220).

Aus diesem Grunde werden als Therapeutika bevorzugt peroral verabreichbare Phosphatbinder eingesetzt, die die Resorption der Nahrungsphosphate im Gastrointestinaltrakt verhindern sollen. Bekannte Substanzen mit phosphatbindenden Eigenschaften sind Calciumsalze (z.B. Calciumacetat, Calciumcarbonat, Calciumcitrat, Calciumalginat, Calciumgluconat, Calciumlactat und Calciumsulfat), Magnesiumcarbonat und Magnesiumhydroxid sowie Aluminiumhydroxid und Aluminiumcarbonat. Nicht alle diese Salze haben jedoch therapeutische Bedeutung erlangt. Gebräuchlich sind Aluminiumhydroxid (z.B. Antiphosphat®, Gry-Pharma GmbH), Calciumcarbonat und Calciumacetat (U.S. Pat. 4,870,105 (1989)). Diese Mittel zur enteralen Phosphatrestriktion besitzen jedoch unerwünschte Nebenwirkungen. So kann sich bei der chronischen Gabe von $Al^{3+}$-Verbindungen eine mikrozytäre Anämie oder eine Enzephalopathie mit sehr schlechter Prognose entwickeln oder es treten Osteopathien auf. Schwerwiegende Nachteile bei einer Langzeittherapie mit Calciumsalzen sind die Entstehung einer ausgeprägten Hypercalcämie, die mit Gefäß- und Weichteilverkalkungen einhergeht, sowie gastrointestinale Beschwerden (Dialyse Journal 37 (1991), 1-40).

Außer diesen salzartigen Phosphatbindern ist aus DE 28 15 811 C2 (1978) ein makroporöses Sorptionsmittel bekannt, das dadurch gekennzeichnet ist, daß es ein organischer Kationenaustauscher ist, der mit Ionen mindestens eines Metalls beladen ist, dessen Phosphat nur schwer löslich ist. Dieses Sorptionsmittel eignet sich jedoch nur für die Entfernung von Phosphat aus proteinfreien Flüssigkeiten (z.B. Dialysierflüssigkeit), da es bei der Verwendung in einem extrakorporalen System zur Elimination von Phosphat aus Vollblut oder Plasma die ionisch gebundenen Metallionen in unerwünschter Weise freisetzt (siehe vergleichendes Beispiel 10).

Weiterhin beschreiben Burt, H.M. et al. (J.Pharm.Sci. 75 (1987), 379-383) Anionenaustauscher auf DOWEX®-Basis, die als funktionelle Gruppe tertiäre oder quartäre Amine tragen und anorganisches Phosphat im Intestinaltrakt adsorbieren. Bekannterweise binden jedoch stark basische Anionenaustauscher wie beispielsweise Cholestyramin (Johns, W.H., Bates, T.R., J.Pharm.Sci. 59 (1970), 788 ff.) unerwünschterweise auch Gallensäuren und führen somit bei einer chronischen Anwendung zu einer Hypovitaminose.

Eine Aufgabe der Erfindung ist es daher, gegenüber dem Stand der Technik verbesserte Adsorbermaterialien zur Verfügung zu stellen, die eine selektive Entfernung von anorganischem Phosphat, das im allgemeinen in Form von $HPO_4^{2-}$ und $H_2PO_4^-$ auftritt, aus wäßrigen Flüssigkeiten und insbesondere aus proteinhaltigen Körperflüssigkeiten, wie beispielsweise Vollblut, Plasma und Darminhalt, ermöglichen. Dabei sollen die übrigen Komponenten der zu behandelnden Flüssigkeiten keine für den Patienten unerwünschte oder schädliche Wechselwirkungen mit den Adsorptionsmaterialien eingehen. Darüber hinaus soll die Bindungskapazität des Adsorbens gegenüber Phosphat optimalen praktischen therapeutischen Anforderungen genügen und das Adsorptionsmaterial muß mit Hitze- oder/und Gamma-Strahlen sterilisierbar sein.

Diese Aufgabe wird gemäß der Erfindung dadurch gelöst, daß man eine in Wasser unlösliche Zusammensetzung, die ein mit polynuklearen Metalloxidhydroxiden modifiziertes Adsorptionsmaterial enthält, als Arzneimittel zur selektiven Elimination von anorganischem Phosphat aus Flüssigkeiten, insbesondere aus proteinhaltigen Körperflüssigkeiten wie Vollblut, Plasma, Darminhaltflüssigkeit sowie Dialysierflüssigkeit verwendet.

Mit polynuklearen Metalloxidhydroxiden modifizierte Adsorptionsmaterialien sind bekannt. Im Journal of Chromatography, Band 481 (1989), 175-199, S. Hjerten und Coautoren, wird die Herstellung von nichtporösen Agarose-Partikeln beschrieben, die mit polynuklearen Metalloxidhydroxiden beschichtet sind. Die Autoren verwenden diese Partikel jedoch nur als chromatographische Trägermaterialien zur analytischen Trennung von Proteingemischen. Zu diesem Zweck werden die Metalloxidhydroxid-beschichteten Agarose-Partikel mit einer Phosphatlösung äquilibriert und dadurch in die für die erwünschte Trennleistung notwendige Metalloxidhydroxid-Phosphat-Spezies überführt. Eine therapeutische Anwendung dieser Adsorptionsmaterialien wird nicht gelehrt.

Bekannt ist weiter die Verwendung von polynuklearen Eisen(III)oxidhydroxid-Polyol-Komplexen als wäßrige Lösungen in der Veterinärmedizin bei der parenteralen Therapie des Eisenmangels. Die Herstellung und Struktur derartiger makromolekularer Eisen(III)oxidhydroxid-Komplexe beschreibt A. Müller (Arzneimittelforschung, Heft 8 (1967), 921-931). Eine therapeutische Anwendung zur selektiven Entfernung von anorganischem Phosphat aus Körperflüssigkeiten wird nicht vorgeschlagen.

Die Verwendung der erfindungsgemäßen Adsorptionsmaterialien besitzt im Gegensatz zu einer Verwendung schon bekannter und oben erwähnter Anionen bzw. Kationenaustauscher den überraschenden Vorteil, daß sie das anorganische Phosphat mit hoher Affinität binden. Die Elimination des Phosphats ist daher unabhängig von dessen Konzentration in der zu behandelnden Flüssigkeit (Beispiele 5, 6, 8, 9). Diese Eigenschaft ist für medizinische Zwecke von Vorteil, da hierdurch eine bislang noch nicht durchführbare, d.h. kontrollierte und therapeutisch adäquate Elimination des anorganischen Phosphats erfolgen kann.

Bei der Herstellung von mit polynuklearen Metalloxidhydroxiden modifizierten Adsorptionsmaterialien zur erfindungsgemäßen Anwendung muß darauf geachtet werden, daß eine Sauerstoffbrückenbildung zwischen verschiedenen Metallatomen eintritt. Zu diesem Zweck kann zuerst entweder das polynukleare Metalloxidhydroxid hergestellt und dann mit dem Basismaterial verbunden werden, oder es wird vorzugsweise auf dem Basismaterial selbst, und zwar durch Tränken des Basismaterials mit einer Suspension oder besonders bevorzugt mit einer Lösung eines geeigneten Metallsalzes und anschließende Erhöhung des pH-Wertes auf stark alkalische Bedingungen (pH $\geq$ 10), gegebenenfalls unter Erhitzen, hergestellt. Für die feste Bindung des Metalls an das Basismaterial ist es wichtig, daß sich auf diesem genügend freie reaktive Gruppen, vorzugsweise organische oder/und anorganische OH-Gruppen, befinden.

Als Ausgangsmaterial zur Herstellung der mit polynuklearen Metalloxidhydroxiden modifizierten Trägermaterialien kann jedes poröse Basismaterial mit einer bevorzugten molekularen Ausschlußgrenze von $10^2$ bis $10^6$ Dalton benutzt werden, besonders bevorzugt beträgt seine molekulare Ausschlußgrenze weniger als $10^4$ Dalton. Vorzugsweise verwendet man jedoch einen solchen Träger, der organische oder/und anorganische Hydroxyl-Funktionalitäten (OH-Gruppen) aufweist. So können beispielsweise organische Träger wie etwa vernetzte Kohlenhydrate, organische Polymerisate oder Copolymerisate, natürliche, halbsynthetische oder synthetische, lineare oder/und verzweigtkettige, lösliche oder unlösliche Polyhydroxy-Verbindungen, z.B. Agarose, Dextran, Dextrin, Cellulose oder/und Polyvinylalkohol als Basismaterial verwendet werden. Spezifische Beispiele sind Trisacryl GF (quervernetztes N-Acryloyl-2-amino-2-hydroxymethyl-1,3-propandiol; Fa. LKB Produkter AB, Bromma, Schweden), TSK-Gele (Copolymere aus Ethylenglykol, Glycidylmethacrylat und Pentaerythroldimethacrylat, z.B. TSK HW 65; Fa. E. Merck, Darmstadt), vernetzte Agarose (z.B. Sepharose; Fa. Pharmacia, Uppsala, Schweden), Cellulose-Gele (z.B. Sephacel, Fa. Pharmacia, Uppsala, Schweden). Andererseits kann man auch anorganische Träger, insbesondere solche auf Siliciumdioxid- oder/und Silikatbasis wie etwa Glyceryl-modifizierte Gläser (z.B. Bioran®-CPG, OH-Modifikation; Fa. Schott Glaswerke, Mainz) und Glyceryl-modifizierte Kieselgele (z.B. LiChroprep-Diol, Fa. E. Merck, Darmstadt) einsetzen.

Erfindungsgemäß bevorzugt eingesetzt werden ein verseiftes Copolymer aus Vinylacetat und Divinylethylen-Harnstoff (VA-Hydroxy Biosynth®, Fa. Riedel de Haen, Seelze) (Beispiel 2), Copolymere der Lewatit R-Reihe (z.B. Lewatit R 249-K; Fa. Bayer AG, Leverkusen) (Beispiel 3) und mit Dextran (Molekulargewicht: 5 x $10^3$ x 5 x $10^6$ Dalton) nach dem Fachmann bekannten Verfahren modifizierte anorganische (Kieselgele, Gläser) oder organische poröse Träger.

Besonders bevorzugte Ausgangsmaterialien sind Dextran-Gele (Beispiel 1), wie beispielsweise die Produkte der Dormagel N® Reihe (Fa. Pfeiffer & Langen, Dormagen), die eine mittlere molekulare Ausschlußgrenze von $10^2$ bis $10^6$, bevorzugter kleiner $10^4$ Dalton besitzen. Die Kopplung an Dextran kann z.B. nach den in Affinity Chromatography, IRL-Press, Oxford (1985) beschriebenen Methoden erfolgen.

Das Basismaterial für das modifizierte Adsorptionsmaterial ist vorzugsweise eine poröse teilchenförmige Substanz, die eine mittlere Korngröße zwischen 5 und 500 µm aufweist.

Zur Verwendung als polynukleare Metalloxidhydroxide ist eine große Anzahl von Metallen geeignet, beispielsweise alle Übergangsmetalle, wie etwa Zirkonium, aber auch Aluminium. Als besonders bevorzugtes Metall wird jedoch Eisen verwendet, da bei einer möglicherweise geringen Ablösung des Metalls Eisen dasjenige Metall ist, das für den Körper als am unschädlichsten anzusehen ist. Aus physiologischen Gründen ist daher dreiwertiges Eisen als Metall am meisten bevorzugt, obgleich auch andere Metalle aufgrund ihrer Bindungseigenschaften bezüglich des anorganischen Phosphats brauchbar sind.

Überraschenderweise zeichnen sich die erfindungsgemäß verwendeten Adsorptionsmaterialien dadurch aus, daß sie das polynukleare, kovalent bzw. koordinativ an den Träger gebundene Metalloxidhydroxid bzw. Metallion, insbesondere die bevorzugterweise verwendete Eisen(III)verbindung auch bei Kontakt mit proteinhaltigen Flüssigkeiten, wie beispielsweise Vollblut und/oder Plasma, nicht in nennenswertem Umfang freisetzen (siehe Beispiele 7 und 10) und somit bei der erfindungsgemäßen therapeutischen extrakorporalen oder/und peroralen Anwendung keine unerwünschten Nebenwirkungen, wie beispielsweise eine Störung der enteralen Eisenresorption oder des zellulären und insbesondere des erythrozytären Eisenstoffwechsels hervorrufen.

Darüber hinaus gehen die erfindungsgemäß verwendeten Adsorptionsmaterialien keine unerwünschten Wechselwirkungen mit den übrigen Komponenten der Körperflüssigkeiten ein und rufen beispielsweise bei Kontakt mit Vollblut keine Aktivierung des Gerinnungssystems oder eine Hämolyse hervor (Beispiele 7, 8). Die erfindungsgemäß verwendeten Adsorptionsmaterialien eignen sich daher in besonders vorteilhafter Weise für die selektive Elimination von anorganischem Phosphat aus Körperflüssigkeiten, wie etwa Plasma oder/und Vollblut in einem extrakorporalen Perfusionssystem bei einer dialysebedingten Hyperphosphatämie oder aus einer Dialysierflüssigkeit. Diese Vollblutverträglichkeit ist vom medizinisch-therapeutischen Standpunkt her von großem Interesse, da hierdurch die Elimination des Phosphats simultan zur Hämodialysebehandlung erfolgen kann (Beispiel 9). Damit entfallen kostspielige und komplexe Vorrichtungen zur Abtrennung und Rückführung der Erythrozyten, und es wird eine wesentliche Vereinfachung und Verbilligung des Verfahrens erreicht. Für die erfindungsgemäß Verwendung in einem extrakorporalen Perfusionssystem werden bevorzugt Trägermaterialien mit einem mittleren Partikeldurchmesser von 100 bis 500 μm, besonders bevorzugt 200 bis 500 μm, eingesetzt.

Die Erfindung betrifft somit ein Verfahren zur selektiven Elimination von anorganischem Phosphat aus Körperflüssigkeiten, wie etwa Vollblut oder/und Plasma in einem extrakorporalen Perfusionssystem oder aus Dialysierflüssigkeit, welches dadurch gekennzeichnet ist, daß die zu behandelnde Flüssigkeit über ein mit einem polynuklearen Metalloxidhydroxid, vorzugsweise Fe(III)oxidhydroxid, modifiziertes Adsorptionsmaterial geleitet wird.

Ein weiterer Gegenstand der Erfindung ist demgemäß eine Vorrichtung zur medizinisch-therapeutischen extrakorporalen Entfernung von anorganischem Phosphat aus wäßrigen Flüssigkeiten, insbesondere Dialysierflüssigkeit, Vollblut oder/und Plasma. Diese Vorrichtung umfaßt ein mit einem Zulauf und einem Ablauf versehenes, vorzugsweise zylindrisches Gehäuse, das mit einem mit polynuklearen Metalloxidhydroxiden modifizierten Adsorptionsmaterial gefüllt ist. Das Gehäuse ist vorzugsweise an seinen stirnseitigen Enden mit Deckeln versehen, die jeweils einen zentralen Zu- bzw. Ablaufstutzen aufweisen (Beispiel 9). Besonders bevorzugt weist dieses zylindrische Gehäuse einen Durchmesser von 3 bis 20 cm, vorzugsweise 5 bis 10 cm und eine Länge von 1 bis 40 cm, vorzugsweise 10 bis 20 cm auf. Das bevorzugte Material für das Gehäuse ist Glas oder Kunststoff.

In dieser erfindungsgemäßen Vorrichtung sind vorzugsweise in die Deckel des zylindrischen Gehäuses Siebe mit 10 bis 300 μm Porenweite zur Eliminierung von Partikeln integriert. Die erfindungsgemäße Vorrichtung ist in einer Verpackung mittels Strahlen (z.B. Gamma-Strahlen) oder Hitze sterilisierbar (Beispiel 4) und damit besonders zum Einsatz in einem extrakorporalen Perfusionssystem oder/und zur Reinigung der Dialysierflüssigkeit geeignet.

Eine weitere Ausführungsform der Erfindung betrifft die Verwendung der Adsorptionsmaterialien in Form oraler Präparate für die enterale Adsorption bzw. Elimination von anorganischem Phosphat. Als besonders vorteilhaft für eine orale Darreichungsform erweist sich die irreversible und damit über die Dosis gut kontrollierbare Bindungseigenschaft bzw. -kapazität, der neutrale Geschmack und die einfache galenische Zubereitung. Zu diesem Zweck werden die erfindungsgemäß verwendeten Adsorptionsmaterialien in einer Korngröße von 5 bis 200 μm, bevorzugterweie 5 bis 20 μm oder/und als Pulver mit Hilfe dem Fachmann bekannter Verfahren verpreßt und mit einer Magensäureresistenten Schicht (z.B. Eudragit L 30 D, Fa. Röhm Pharma, Weiterstadt) überzogen oder in säurestabile Kapseln gefüllt.

Ein weiterer Gegenstand der vorliegenden Erfindung ist somit ein Verfahren zur Herstellung eines oral zu verabreichenden Arzneimittels für die selektive Entfernung von anorganischem Phosphat, welches dadurch gekennzeichnet ist, daß man ein mit polynuklearen Metallhydroxiden modifiziertes Adsorptionsmaterial als solches oder in Pulverform verpreßt mit einer Magensaft-resistenten Schicht überzieht oder in eine säurestabile Kapsel füllt.

Für die erfindungsgemäße perorale Verwendung werden bevorzugt Trägermaterialien mit einem mittleren Partikeldurchmesser von 5 bis 200 μm, vorzugsweise 5 bis 20 μm, eingesetzt.

Für die erfindungsgemäße perorale Verwendung polynuklearer Metalloxidhydroxid-Polyol Komplexe können auch die entsprechenden Polyol-Grundbausteine der vernetzten Polysaccharid-Träger, wie beispielsweise Agarose, Dextran, Dextrin, Dextranderivate, Cellulose und Cellulosederivate, verwendet werden.

Die chemische Modifikation der aufgeführten Adsorbentien wird am Beispiel der mit Eisen(III)salzen bevorzugten Ausführungsform erläutert (siehe Herstellungsbeispiele 1-3: Dreiwertiges Eisen bindet als Zentralatom in wäßriger Lösung koordinativ sechs Wassermoleküle. Ein oder mehrere dieser Wassermoleküle werden unter stark alkalischen Bedingungen (pH größer als 10) gegen eine funktionelle OH-Gruppe des Trägermaterials ausgetauscht. Dieser Vorgang führt zur koordinativen Bindung der Eisen(III)-Aquo-Komplexe auf der Trägeroberfläche. Darüber hinaus begünstigt das stark alkalische Milieu die Deprotonierung des am Eisen-Zentralatom koordinativ gebundenen Wassers. Die deprotonierten Wassermoleküle bilden sodann $O^{2-}$-Brücken zwischen zwei benachbarten Eisen-Zentralatomen. Dieser Prozeß führt zu einer dreidimensionalen Verknüpfung der Eisen-Aquo-Komplexe. Es entsteht ein auf der Trägeroberfläche gebundenes Netzwerk aus polynuklearen (mehrkernigen) Eisen(III)oxidhydroxid-Komplexen.

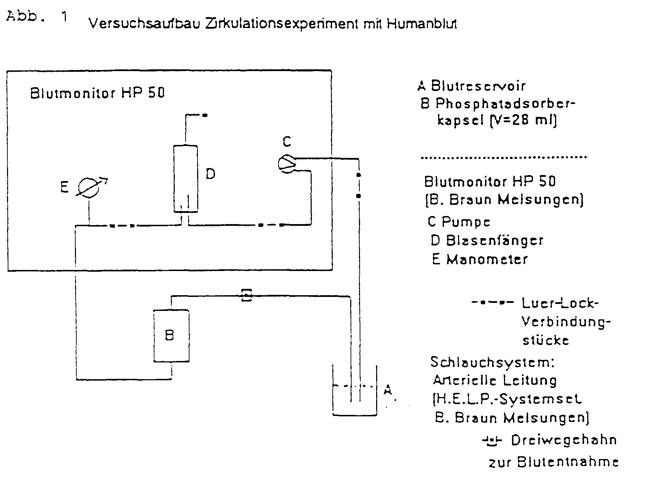

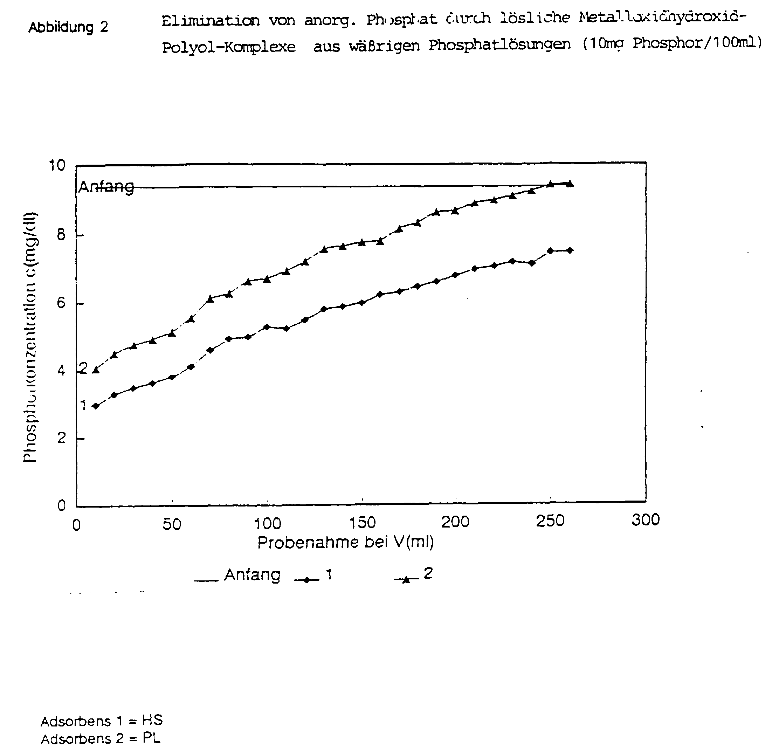

Die Erfindung wird durch die folgenden Beispiele in Verbindung mit den Abbildungen 1 und 2 näher erläutert.

Abb. 1    zeigt das Schema einer Anordnung zur Entfernung von anorganischem Phosphat aus Humanblut.

Abb. 2    zeigt die Elimination von anorg. Phosphat durch lösliche Metalloxidhydroxid-Polyol-Komplexe aus wäßrigen Phosphatlösungen.

## Beispiel 1

Synthese von Dextran-Eisen(III)-Komplexträger

40 g getrockneter Dextranträger (Fa. Pfeiffer & Langen Dormagel N®25 C) werden unter Rühren (Flügelrührer 75 rpm) mit 200 ml Eisen(III)chloridlösung (Merck, Darmstadt, 100 g $FeCl_3$ x $6H_2O$ mit bidest. Wasser auf 200 ml Volumen eingestellt, entspricht 50 % Lösung) versetzt. Die Suspension wird ca. 12 Std. einem Quellprozeß unterzogen, anschließend wird der Ansatz in 2 L 0,7 N Natronlauge unter starkem Rühren (Flügelrührer 500 rpm) eingebracht. Nach 15 min Rührprozeß wird mit entionisiertem Wasser bis pH 9 gespült. Nach 5-minütiger Ultraschallbehandlung wird erneut mit entionisiertem Wasser bis zum pH-Wert 7,5 gespült.

Der Gehalt der Trägermaterialien an Eisen bzw. Eisen(III)oxidhydroxid-Komplexen wird über Atomabsorptionsspektroskopie bzw. Emissionsspektroskopie mit induktiv gekoppeltem Plasma ermittelt.

Der Eisengehalt der Träger kann über die Menge an Eisen(III)chlorid oder/und über die Anzahl der Umsätze entsprechend obiger Synthesevorschrift (siehe Tabelle 1) gezielt und reproduzierbar verändert werden.

Tabelle 1

| Eisengehalt, bezogen auf Trockengewicht | |
|---|---|
| | Fe-Gehalt (%) |
| Einmaliger Umsatz | 15,7 |
| Zweimaliger Umsatz | 25,5 |
| Dreimaliger Umsatz | 29,3 |

## Beispiel 2

Synthese von Vinylacetat-Copolymerisat-Eisen(III)-Komplexträger

10 g getrockneter Vinylacetat-Hydroxycopolymer-Träger (Biosynth; Riedel de Haen) werden unter manuellem Rühren mit 20 ml einer 50 % (w/w) Eisen(III)hexahydratlösung (Merck, Darmstadt) vermischt. Der Quellvorgang der Suspension beläuft sich auf ca. 1 Stunde, anschließend wird der Syntheseansatz einer 5-minütigen Ultraschallbehandlung unterzogen. Unter starkem Rühren (Flügelrührer 500 rpm) wird der Ansatz in 200 ml 1 N Natronlauge eingebracht. Anschließend wird der Eisen(III)oxidhydroxid-modifizierte Träger mit entionisiertem Wasser bis pH-Wert 9 gespült, erneut mit Ultraschall behandelt (5 min) und mit entionisiertem Wasser durch Spülen auf den pH-Wert 7,5 eingestellt.

Der Gehalt an Eisen(III) beträgt 17 % (ermittelt über Atomabsorptionsspektroskopie).

## Beispiel 3

Synthese von Divinylbenzolcopolymer-Eisen(III)-Komplexträger

50 g getrocknetes Divinylbenzolcopolymer (Lewatit R 1836/249/257-260, Fa. Bayer, Leverkusen) werden in die Hydroxid-aktivierte Form überführt (mit zweifachem "Batchvolumen" 96 % Ethanol (Fa. Merck, Darmstadt) 2 Stunden schütteln, nach Filtration mit zweifachem "Batchvolumen" 2 N-Schwefelsäure (Fa. Merck, Darmstadt) versetzen und nach Filtration waschen). Der Hydroxy-aktivierte Träger (50 g) wird mit 50 g Eisen(III)chloridhexahydrat und 5 ml bidest. Wasser versetzt und manuell bis zur Homogenität verrührt. Nach 5-minütiger Ultraschallbehandlung wird die Suspension in 750 ml 1 N Natronlauge eingebracht. Nach 15-minütigem Rühren (Flügelrührer 500 rpm) wird der Träger mit entionisisertem Wasser bis zum pH 9 gespült, mit Ultraschall behandelt (5 min) und erneut bis zu einem pH von 7,5 gespült.

Der Eisen(III)-Gehalt der Träger beträgt je nach Spezies zwischen 6,4 - 10 % Eisen (gemessen über Atomabsorptionsspektroskopie).

**Beispiel 4**

Sterilisation der Eisen(III)oxidhydroxid-modifizierten Trägermaterialien

Adsorbens:

1. Dextran-Eisen(III)-Komplexträger (DE)
2. Vinylacetat-Copolymerisat-Eisen(III)-Komplexträger (VA)
3. Divinylbenzolcopolymer Eisen(III)-Komplexträger (DVB Typ 260)

Die Adsorbermaterialien 1-3 werden nach der Synthese im Meßzylinder auf 50 ml Volumen eingestellt und anschließend mit 100 ml Wasser in einer mit Gummistopfen verschließbaren Glasflasche überführt und unter $F_{015}$-Hitzesterilisationskondition sterilisiert.

Parallel werden die Adsorbermaterialien im Trockenschrank bei 60°C getrocknet, in Alufolien unter Vakuum eingeschweißt und ebenfalls unter $F_{015}$-Kondition hitzesterilisiert.

Vor und nach Sterilisation wird der Eisengehalt überprüft. Die Ergebnisse sind in Tabelle 2 dargestellt. Durch den Sterilisationsprozeß wird in keinem Fall der Eisengehalt der Träger beeinträchtigt.

Tabelle 2

| Eisengehalt der Trägermaterialien vor und nach Sterilisation | | | | |
|---|---|---|---|---|
| | Eisengehalt (%)bezogen auf Trockengewicht | | | |
| Adsorbens | vor Sterilisation wäßriges Produkt | nach Sterilisation wäßriges Produkt | vor Sterilisation trock. Produkt | nach Sterilisation trock. Produkt |
| 1 | 20,1 | 20,1 | 20 | 19,9 |
| 2 | 17,3 | 17,2 | 17,3 | 17,4 |
| 3 | 6,4 | 6,2 | 6,3 | 6,3 |

**Beispiel 5**

Bindungskapazität der Trägermaterialien gemäß den Beispielen 1-3 für anorganisches Phosphat aus einer wäßrigen Phosphatlösung und phosphathaltiger Dialysierflüssigkeit

Adsorbens:

1. Dextran-Eisen(III)-Komplexträger (DE)
2. Vinylacetat-Copolymerisat-Eisen(III)-Komplexträger (VA)
3. Ester-modifizierter Divinylbenzolcopolymer Eisen(III)-Komplexträger (DVB Typ 260)

Versuchsdurchführung:

A. Wäßrige Phosphatlösung (10 mg Phosphor/100 ml) Herstellung:

5,8 g/l NaCl = 100 mmol/l
0,29 g/l KCl = 4 mmol/l
0,07 g/l $Na_2SO_4$ = 0,5 mmol/l
0,28 g/l $Na_2HPO_4$ x $2H_2O$ = 1,6 mmol/l
0,24 g/l $NaH_2PO_4$ x $2H_2O$ = 1,6 mmol/l

B: Dialysierflüssigkeit: HDY 314 (B. Braun Melsungen AG) mit je 1,6 mmol $Na_2HPO_4$ x $2H_2O$ und $NaH_2PO_4$ x $2H_2O$ versetzt.

Die Adsorbermaterialien (1-3) werden mit bidest. Wasser über eine G3 Nutsche gewaschen, in eine Chromatographiesäule (Biorad 120 mm x 10 mm) gefüllt und anschließend mit 300 ml 50 mmol/l Tris-HCl pH 7,4 äquilibriert (Säulenbettvolumen: 3 ml).

Auf die Säule werden bei Raumtemperatur 350 ml der Lösungen A oder B gegeben und durch die Säule gepumpt (Volumenstrom 1 ml/min). Nach 4 ml Voreluat wurden jeweils Fraktionen von 10 ml gewonnen und deren Phosphat- bzw. Phosphorgehalt mittels eines photometrischen Phosphor-Molybdän-Tests ermittelt.

Das Phosphatbindungsvermögen in % errechnet sich aus:

$$\text{Phosphatbindungsvermögen} = \frac{\text{Ausgangskonz. - Gesamteluatkonz. x 100}}{\text{Ausgangskonzentration}}$$

Tabelle 3

| Phosphatbindung der Adsorbentien | | | | |
|---|---|---|---|---|
| Adsorbens | (%) | mg Phosphat/ ml | mg Phosphor/ml | mmol Phosphat/ml |
| wäßrige Phosphatlösung (A) | | | | |
| 1 | 22 | 7,58 | 2,52 | 0,081 |
| 2 | 19 | 6,65 | 2,21 | 0,071 |
| 3 | 6 | 2,1 | 0,70 | 0,022 |
| Phosphathaltige Dialysierflüssigkeit (B) | | | | |
| 1 | 21 | 7,35 | 2,45 | 0,079 |
| 2 | 19 | 6,65 | 2,21 | 0,071 |
| 3 | 7 | 2,45 | 0,81 | 0,026 |
| Umrechnungsfaktor: mg Phosphor in mg Phosphat = 3,161 | | | | |

**Beispiel 6**

Bindungsvermögen der Trägermaterialien für anorganisches Phosphat aus Humanplasma

Adsorbens:

1. Dextran-Eisen(III)-Komplexträger (DE)
2. Vinylacetat-Copolymerisat-Eisen(III)-Komplexträger (VA)
3. Divinylbenzolcopolymer Eisen(III)-Komplexträger (DVB Typ 260)
4. Divinylbenzolcopolymer-Eisen(III)-Komplexträger (DVB Typ 1836/88, Korngrößenverteilung 100-250 μm)
5. Divinylbenzolcopolymer-Eisen(III)-Komplexträger (DVB Typ R 249, Korngrößenverteilung 200-500 μm)

Versuchsdurchführung:

Analog Beispiel 5. 100 ml Humanplasma werden mit 3 Einheiten Na-Heparin (B. Braun Melsungen AG) pro ml stabilisiert und über die Säule gepumpt. In Tabelle 4 sind die Phosphatadsorptionskapazitäten der eingesetzten Trägerspezies aufgeführt.

Tabelle 4

| Bindungsvermögen von Phosphat aus Humanplasma | | | |
|---|---|---|---|
| Adsorbens | % | mg Phosphor/ml | mmol Phosphat/ml |
| 1 DE | 56 | 2,1 | 0,067 |
| 2 VA | 65 | 2,4 | 0,077 |
| 3 Typ 260 | 26 | 1,0 | 0,032 |
| 4 Typ 1836/88 | 90 | 3,4 | 0,109 |
| 5 Typ 249 | 50 | 1,9 | 0,061 |
| Umrechnungsfaktor: mg Phosphor in mg Phoshat = 3,161 | | | |

**Beispiel 7**

Selektivität der Adsorbentien bezüglich der Eliminierung von anorganischem Phosphat aus Humanplasma

Adsorbens:

1. Dextran-Eisen(III)-Komplexträger (DE)
2. Divinylbenzolcopolymer-Eisen(III)-Komplexträger (DVB Typ R 249)

Versuchsdurchführung:

Analog Beispiel 6. Für die Analytik werden nach 4 ml Totvolumen jeweils 10 Eluatfraktionen a 10 ml gewonnen, die dann auf die klinisch-chemisch relevanten Parameter des Humanplasmas untersucht werden.

Die Ergebnisse dieses Experiments sind in Tabelle 5 für Adsorbens 1 und Tabelle 6 für Adsorbens 4 dargestellt.

Tabelle 5

| Klinisch-chemische Kenngrößen vor und nach der Säulenpassage von Humanplasma über einen Dextran-Eisen(III)-Komplexträger (DE N25; gemäß Beispiel 1) | | | | | | | | | | | | |
|---|---|---|---|---|---|---|---|---|---|---|---|---|
| Parameter | E1 | E2 | E3 | E4 | ES | E6 | E7 | E8 | E9 | E10 | Anf. | Konz. |
| Magnesium | 1,76 | 1,85 | 1,92 | 1,94 | 1,93 | 1,95 | 1,94 | 1,99 | 1,96 | 1,96 | 1,99 | mg/dl |
| Phosphor | 0,9 | 1,7 | 2,2 | 2,6 | 2,8 | 2,9 | 3,1 | 3,1 | 3,2 | 3,2 | 3,3 | mg/dl |
| Natrium | 143,4 | 143,7 | 143,0 | 143,7 | 143,0 | 144,0 | 143,8 | 143,7 | 143,5 | 144,5 | 143,7 | mmol/l |
| Kalium | 3,44 | 3,51 | 3,52 | 3,54 | 3,52 | 3,54 | 3,52 | 3,52 | 3,51 | 3,54 | 3,52 | mmol/l |
| Calzium | 1,64 | 1,91 | 1,98 | 2,02 | 2,06 | 2,05 | 2,07 | 2,09 | 2,09 | 2,08 | 2,18 | mmol/l |
| Chlorid | 106,8 | 105,8 | 105,7 | 106,5 | 106,0 | 106,5 | 106,3 | 106,5 | 106,3 | 106,5 | 105,8 | mmol/l |
| Eisen | 98 | 99 | 98 | 98 | 98 | 97 | 98 | 98 | 98 | 98 | 96 | µg/dl |
| Ferritin | 302,6 | 248,8 | 295,9 | 247,0 | 233,0 | 298,2 | 259,4 | 259,4 | 256,4 | 297,5 | 275,3 | ng/ml |
| Transferrin | 279 | 277 | 282 | 281 | 273 | 280 | 281 | 274 | 251 | 276 | 283 | mg/dl |
| Gesamteiweiß | 7,33 | 7,58 | 7,46 | 7,49 | 7,45 | 7,41 | 7,54 | 7,51 | 7,42 | 7,53 | 7,56 | g/dl |
| Kreatinin | 0,87 | 0,92 | 0,93 | 0,91 | 0,92 | 0,94 | 0,93 | 0,94 | 0,91 | 0,94 | 0,90 | mg/dl |
| Albumin | 3890 | 4110 | 3520 | 3700 | 3800 | 3940 | 3630 | 4030 | 3730 | 3720 | 3870 | mg/dl |
| IgG | 1190 | 1270 | 1300 | 1360 | 1300 | 1250 | 1330 | 1260 | 1310 | 1300 | 1260 | mg/dl |
| IgA | 296 | 295 | 262 | 297 | 306 | 302 | 308 | 312 | 312 | 307 | 312 | mg/dl |
| IgM | 114 | 119 | 118 | 121 | 114 | 126 | 119 | 115 | 122 | 114 | 122 | mg/dl |
| mit E1-E10 = Eluat 1 - Eluat 10<br>Anf. = Anfangswert<br>Konz. = Konzentration<br>Umrechnungsfaktor: mg Phosphor in mg Phosphat = 3,161 | | | | | | | | | | | | |

Tabelle 6

| Parameter | E1 | E2 | E3 | E4 | E5 | E6 | E7 | E8 | E9 | E10 | Anf. | Konz. |
|---|---|---|---|---|---|---|---|---|---|---|---|---|
| Klinisch-chemische Kenngrößen vor und nach der Säulenpassage von Humanplasma über einen Divinylbenzol-Eisen(III)-Komplexträger (DVB Typ R 249; gemäß Beispiel 3) | | | | | | | | | | | | |
| Magnesium | 0,17 | 0,17 | 0,24 | 0,38 | 0,56 | 0,80 | 0,99 | 1,22 | 1,35 | 1,454 | 1,99 | mg/dl |
| Phosphor | 0,2 | 0,3 | 0,8 | 1,4 | 2,0 | 2,5 | 2,8 | 3,0 | 3,1 | 3,1 | 3,3 | mg/dl |
| Natrium | 142,7 | 142,8 | 142,3 | 143,1 | 143,7 | 143,8 | 143,0 | 143,5 | 144,2 | 143,1 | 143,7 | mmol/l |
| Kalium | 3,23 | 3,50 | 3,48 | 3,52 | 3,53 | 3,54 | 3,51 | 3,53 | 3,54 | 3,50 | 3,52 | mmol/l |
| Calzium | 2,67 | 2,59 | 2,5 | 2,49 | 5,48 | 2,49 | 2,46 | 2,39 | 2,63 | 2,33 | 2,18 | mmol/l |
| Chlorid | 106,2 | 105,8 | 105,3 | 106,1 | 106,2 | 106,2 | 106,3 | 105,8 | 106,5 | 105,9 | 105,8 | mmol/l |
| Eisen | 99 | 100 | 99 | 99 | 100 | 99 | 98 | 98 | 99 | 100 | 96 | µg/dl |
| Ferntin | 267,1 | 322,5 | 285,8 | 260,0 | 329,6 | 304,7 | 322.8 | 249,1 | 269,0 | 315,8 | 275,3 | ng/ml |
| Transferrin | 277 | 294 | 293 | 277 | 266 | 287 | 255 | 269 | 278 | 281 | 283 | mg/dl |
| Gesamteiweiß | 7,31 | 7,39 | 7,39 | 7,41 | 7,39 | 7,45 | 7,51 | 7,45 | 7,57 | 7,47 | 7,56 | g/dl |
| Kreatinin | 0,90 | 0,90 | 0,88 | 0,91 | 0,89 | 0,90 | 0,89 | 0,91 | 0,94 | 0,90 | 0,90 | mg/dl |
| Albumin | 3780 | 3870 | 4010 | 3960 | 3680 | 3380 | 3790 | 3900 | 3380 | 3560 | 3870 | mg/dl |
| IgG | 1320 | 1290 | 1320 | 1340 | 1330 | 1280 | 1300 | 1290 | 1350 | 1250 | 1260 | mg/dl |
| IgA | 297 | 310 | 305 | 314 | 306 | 290 | 304 | 304 | 309 | 304 | 312 | mg/dl |
| IgM | 119 | 124 | 114 | 123 | 118 | 125 | 118 | 118 | 124 | 121 | 122 | mg/dl |

mit E1-E10 = Eluat 1 - Eluat 10 Anf. = Anfangswert Konz.= Konzentration Umrechnungsfaktor: mg Phosphor in mg Phosphat = 3,161

**Beispiel 8**

Blutverträglichkeit - Hämokompatibilität der Adsorbentien

Adsorbens:

1. Dextran-Eisen(III)-Komplexträger (DE N25c; gemäß Beispiel 1)

Versuchsdurchführung:

Phosphat-Elimination aus heparinisiertem Vollblut

Eine 35 ml Kartusche, die an beiden Enden mit einem 100 µm-Filter versehen ist, wird mit Adsorbens 1 gepackt, mit bidest. Wasser (500 ml) und anschließend mit Ringerlösung (5 IE/ml Na-Heparin, B. Braun Melsungen) gespült. Danach wird frisch gewonnenes, heparinisiertes Vollblut (4 IE/ml Heparin) durch hydrostatischen Druck über die Kartusche bei einem Fluß von 20 ml/min 530 ml geleitet und 12 Fraktionen mit je 45 ml gewonnen und auf deren Blutbild untersucht. In den Tabellen 7, 8 und 9 sind die Ergebnisse dieses Experiments dargestellt.

Tabelle 7

| Blutbild (automatische Zellzählung) | | | | | | | |
|---|---|---|---|---|---|---|---|
| Fraktion (45 ml) | 2 | 4 | 6 | 8 | 10 | Ausgangswert | Dimension |
| Leukozyten | 5,2 | 5,5 | 5,4 | 4,9 | 5,1 | 6,5 | $10^9$/l |
| Lymphozyten | 61,5 | 59,4 | 60,1 | 65,6 | 62,8 | 63,4 | % |
| Monozyten | 3,9 | 4,1 | 4,2 | 3,8 | 4,9 | 3,9 | % |
| Eosinophile | 6,1 | 6,8 | 6,2 | 6,2 | 6,3 | 6,2 | % |
| Basophile | 0,3 | 0,0 | 0,0 | 2,8 | 0,1 | 0,2 | % |
| Erythrozyten | 4,51 | 4,54 | 4,47 | 4,50 | 4,51 | 4,25 | $10^3$/l |
| Hämoglobin | 15,2 | 15,3 | 15,3 | 15,3 | 15,3 | 14,3 | g/dl |
| Hämatokrit | 43,1 | 43,1 | 43,8 | 42,5 | 43,1 | 40,7 | % |
| mittleres corpuskuläres Volumen (MCV) | 95,5 | 96,3 | 95,0 | 95,7 | 95,4 | 95,7 | fl |
| mittleres corpuskuläres Hämoglobin (MCH) | 35,7 | 33,8 | 34,1 | 34,0 | 34,0 | 33,6 | pg |
| mittlere corpuskuläre Hämoglobinkonz. (MCHC) | 35,3 | 35,0 | 35,9 | 35,5 | 35,6 | 35,0 | g/dl |
| Thrombozyten | 122 | 122 | 134 | 89 | 91 | 226 | $10^9$/l |
| mittleres Thrombozytenvolumen | 8,3 | 8,2 | 8,3 | 8,0 | 8,2 | 8,6 | fl |
| freies Hämoglobin | 1,95 | 2,01 | 2,19 | 2,20 | 2,15 | 1,55 | mg/dl |

Tabelle 8

| Differentialblutausstrich | | | |
|---|---|---|---|
|  | Ausgangswert | Fraktion 4 | Dimension |
| Lymphozyten | 71 | 74 | % |
| Monozyten | 1 | 4 | % |
| Eosinophile | 4 | 3 | % |
| Basophile | 5 | 1 | % |
| Stabkernige | 0 | 2 | % |
| Segmentkernige | 19 | 16 | % |

Tabelle 9

| Gruppentest der plastischen Gerinnung | | | |
|---|---|---|---|
| | Ausgangswert | Fraktion 4 | Dimension |
| Fibrinogen | 376 | 370 | mg/dl |
| Plasminogen | 119 | 118 | % |
| Antithrombin III | 105 | 102 | % |
| Partielle Thromboplastinzeit (PTT) | 33 | 37 | sec. |
| Thromboplastinzeit (Quick-Wert) | 100 | 100 | % |

Der Vergleich der Kenngrößen vor und nach Kontakt des Plasmas bzw. Blutes mit dem erfindungsgemäßen Adsorptionsmaterial zeigt, daß das Gerinnungssystem nicht in unerwünschter Weise durch die Behandlung beeinträchtigt wird.

**Beispiel 9**

In vitro Zirkulationsexperiment von Humanblut über ein pumpenbetriebenes Kartuschensystem

Ziel des Experiments ist die Überprüfung der strömungsmechanischen Belastbarkeit und des Druckverhaltens von Humanblut, das mehrfach rezirkulierend über ein mit dem erfindungsgemäßen Phosphatadsorber gemäß Beispiel 1 und 2 gefülltes Kartuschensystem geleitet wird.

Adsorbens:

1. Dextran-Eisen(III)-Komplexträger (DE)
2. Vinylacetat-Copolymerisat-Eisen(III)-Komplexträger (VA)

Versuchsdurchführung:

Eine zylinderförmige Kapsel (Durchmesser 55 mm, Volumen 28 ml mit integriertem Siebgewebe - Maschenweite 92 µm) wird jeweils mit hitzesterilisierten Adsorbens 1 oder 2 beladen und mit 1,5 l 50 mmol/l Tris-HCl Puffer pH 7,4 äquilibriert (50 ml/min, 20 mm Hg). Nach Spülung des Systems mit 1,5 l physiologischer Kochsalzlösung werden 350 ml Humanblut, die mit 6 Einheiten Na-Heparin (B.Braun Melsungen AG) pro ml versetzt sind, in das Zirkulationsverfahren (siehe Skizze 1) eingeleitet. Das gesamte vorgelegte Blutvolumen wird jeweils kontinuierlich in Austauschzyklen bis zum Eintritt einer Hämolyse über die Kartusche bzw. durch das Schlauchleitungssystem mit einem Volumenstrom von 50 ml/min gepumpt. Die Probenahme (1 ml Humanblut) erfolgt über einen Dreiwegehahn (siehe Abb. 1) nach jedem Austauschzyklus.

Die gewonnenen Blutfraktionen werden auf ihren Phosphatgehalt sowie auf ihre Hämolyseaktivität untersucht. Die Ergebnisse des Experimentes sind in Tabelle 10 dargestellt. Nach 5 bzw. 25 Austauschzyklen überschreiten die Werte der Eluate den Grenzwert der Hämodialyse DIN 58352 von einer Extinktion von $E \geq 0,03$. Das Adsorbens 2 besitzt eine Korngrößenverteilung von 50-200 µm, die sich als nachteilig für die strömungsmechanische Belastbarkeit und das Druckverhalten von Humanblut erweist.

Tabelle 10

| Zirkulationsexperiment mit Humanblut | | | |
|---|---|---|---|
| Material | Phosphatadsorption (%) | Druck mm Hg | Anzahl Austauschzyklen bis Eintritt von Hämolyse |
| Adsorbens 1: Dextranträger 150-300 µm | 90 | 44 | 25 |
| Adsorbens 2: VA-Träger 50-200 µm | 90 | 50 | 5 |

**Beispiel 10**

Vergleichende Untersuchung zur Freisetzung von Eisen(III)-Ionen aus Adsorptionsmaterialien durch proteinhaltige Flüssigkeiten

Adsorbens:

1. gemäß Beispiel 1
2. Eisen(III)-beladener Iminodiessigsäure-Kationenaustauscher (Lewatit R 251 K, Fa. Bayer AG, Leverkusen) gemäß Stand der Technik

Das mit Iminodiessigsäure funktionalisierte Copolymer aus Styrol und Divinylbenzol entspricht dem Sorptionsmittel Nr. 1 in DE 28 15 811. Der Kationenaustauscher wurde zunächst mit 1 N HCl in die $H^+$-Form überführt, mit einer 50 mmol/l Eisen(III)chlorid-Lösung gesättigt und abschließend mit bidest. Wasser gewaschen.

Versuchsdurchführung:

Auf eine mit dem jeweiligen Adsorbens gepackte (2 ml Säulenbettvolumen) und mit einer Ringer-Lösung äquilibrierte Säule werden 10 ml Humanserum gegeben. Die erste 1 ml Fraktion wird verworfen (Verdünnungseffekte) und in den restlichen 1 ml Fraktionen wird der Gehalt an Eisen bestimmt.

Tabelle 11

| Freisetzung von Eisen(III)-Ionen | | |
|---|---|---|
| | Adsorbens 1 | Adsorbens 2 |
| Eisen (µg/100 ml) Ausgangswert | 60 | 60 |
| Eisen (µg/100 ml) Fraktion 1 | 56 | 165 |
| 2 | 62 | 170 |
| 3 | 65 | 183 |
| 4 | 60 | 198 |
| 5 | 63 | 202 |
| 6 | 66 | 214 |
| 7 | 59 | 225 |
| 8 | 66 | 245 |
| 9 | 65 | 274 |

Die erhaltenen Werte zeigen eindeutig eine unerwünschte Freisetzung von Eisen(III)-Ionen durch Serumproteine bei Verwendung eines Adsorbens gemäß DE 28 15 811, während bei dem erfindungsgemäßen Adsorbens keine signifikante Freisetzung von Eisen(III)-Ionen stattfindet.

Beispiel 11 (Vergleich)

Elimination von anorg. Phosphat durch lösliche Metalloxidhydroxid/Polyol-Komplexe aus wässrigen Phosphatlösungen

Adsorbens:

1. Ferrum®-Saft (Fa. Hausmann, St. Gallen) (HS)
2. Dormapher®-Lösung (Fa. Pfeiffer-Langen, Dormagen) (PL)

Versuchsdurchführung:

Wäßrige Phosphatlösung (10 mg Phosphor/100 ml)

Herstellung:

5,8 g/l NaCl = 100 mmol/l
0,29 g/l KCl = 4 mmol/l
0,07 g/l $Na_2SO_4$ = 0,5 mmol/l
0,28 g/l $Na_2HPO_4$ x $2H_2O$ = 1,6 mmol/l
0,24 g/l $NaH_2PO_4$ x $2H_2O$ = 1,6 mmol/l

Je 40 ml des Adsorbens 1 und 2 werden jeweils in eine Ultrafiltrationseinheit (Rührzelle 8200/Amicon, Druck 3 bar) gefüllt und kontinuierlich mit obiger Phosphatlösung versetzt. Die die Phosphationen adsorbierenden Metalloxidhydroxid-Polyol-Komplexe werden durch die Membran (Filtron Omega NM BL 3K) zurückgehalten. Die eluierte Lösung wird nach 40 ml Voreluat in 10 ml Fraktionen gesammelt und deren Phoshat-/Phosphorkonzentration gemäß Beispiel 5 bestimmt. Abb. 2 zeigt die erhaltene Elimination von anorg. Phosphat durch lösliche Metalloxidhydroxid/Polyol-Komplexe aus wäßrigen Phosphatlösungen.

Beispiel 12 (Vergleich)

Bindungskapazität von löslichen Metalloxidhydroxid-Polyol-Komplexen für anorganisches Phosphat aus standardisiertem Darminhalt

Adsorbens:

1. Ferrum®-Saft (Fa. Hausmann, St. Gallen) (HS)
2. Ferrum®-Tropfen (Fa. Hausmann, St. Gallen) (HT)
3. Dormapher®-Lösung (Fa. Pfeiffer-Langen, Dormagen) (PL)

Versuchsdurchführung:

Standardisierter Darminhalt

Herstellung:

6,8 g/l $KH_2PO_4$ = 50 mmol/l
10 g/l Pankreatin
pH 7,5

Je 50 ml der Adsorbentien 1-3 werden mit je 1 Liter obiger Lösung vermischt, in Dialyseschläuche (NMWL 1K Spetropor) gefüllt, 72 Stunden gegen Wasser dialysiert und die Phosphat/Phosphorkonzentration gemäß Beispiel 5 im Dialysat bestimmt.

Tabelle 12

| Phosphoradsorption von löslichen Metalloxidhydroxid-Polyol-Komplexen aus standardisiertem Darminhalt | | |
|---|---|---|
| Adsorbens | gebundene Phosphatmenge (mg) | gebundene Phosphormenge (mg)/ml Adsorbens |
| 1 (HS) | 6 | 0,12 |
| 2 (HT) | 38 | 0,76 |
| 3 (PL) | 16,5 | 0,33 |

**Patentansprüche**

1. Zusammensetzung zur Verwendung als Arzneimittel zur selektiven Elimination von anorganischem Phosphat aus Flüssigkeiten, wobei die Zusammensetzung in Wasser unlöslich ist und ein mit polynuklearen Metalloxidhydroxiden modifiziertes Adsorptionsmaterial enthält.

2. Zusammensetzung nach Anspruch 1,
   **dadurch gekennzeichnet,**
   daß das Basismaterial für das modifizierte Adsorptionsmaterial organische oder/und anorganische Hydroxylgruppen aufweist.

3. Zusammensetzung nach Anspruch 1 oder 2,
   **dadurch gekennzeichnet,**
   daß das Basismaterial für das modifizierte Adsorptionsmaterial eine poröse Struktur mit einer molekularen Ausschlußgrenze von $10^2$ bis $10^6$ Dalton besitzt.

4. Zusammensetzung nach Anspruch 3,
   **dadurch gekennzeichnet,**
   daß die molekulare Ausschlußgrenze weniger als $10^4$ Dalton beträgt.

5. Zusammensetzung nach einem der Ansprüche 1 bis 4,
   **dadurch gekennzeichnet,**
   daß das poröse Basismaterial für das modifizierte Adsorptionsmaterial eine mittlere Korngröße zwischen 5 und 500 μm aufweist.

6. Zusammensetzung nach einem der Ansprüche 1 bis 5,
   **dadurch gekennzeichnet,**
   daß das Basismaterial eine natürliche, halbsynthetische oder synthetische, lineare oder/und verzweigtkettige lösliche oder unlösliche Polyhydroxyverbindung ist.

7. Zusammensetzung nach einem der Ansprüche 1 bis 6,
   **dadurch gekennzeichnet,**
   daß das modifizierte Adsorptionsmaterial ein poröses Basismaterial aus der Gruppe, bestehend aus Materialien auf Silikat- oder Siliciumdioxidbasis, insbesondereGlyceryl-modifizierten Kieselgelen,Glyceryl-modifizierten Gläsern (CPG), vernetzten Kohlenhydraten, organischen Polymerisaten oder Copolymerisaten, aufweist.

8. Zusammensetzung nach einem der Ansprüche 1 bis 7,
   **dadurch gekennzeichnet,**
   daß das Basismaterial aus porösen Kieselgelen, organischen Polymerisaten oder Copolymerisaten besteht, welches mit Polysaccharid, insbesondere Dextran modifiziert ist.

9. Zusammensetzung nach einem der Ansprüche 1 bis 8,
   **dadurch gekennzeichnet,**
   daß das Basismaterial ausgewählt ist aus Agarose, Dextran, Dextrin, Cellulose oder/und Polyvinylalkohol.

10. Zusammensetzung nach einem der vorhergehenden Ansprüche,
    **dadurch gekennzeichnet,**
    daß das Adsorptionsmaterial mit $Fe^{III}$-Oxidhydroxid in polynuklearer Form modifiziert ist.

11. Zusammensetzung nach einem der vorhergehenden Ansprüche,
    **dadurch gekennzeichnet,**
    daß sie in einer oral zu verabreichenden Form ist.

12. Zusammensetzung nach einem der Ansprüche 1 bis 10,
    **dadurch gekennzeichnet,**
    daß sie Bestandteil eines extrakorporealen Perfusionssystems ist.

13. Verwendung eines Adsorptionsmaterials wie in einem der vorhergehenden Ansprüche definiert, welches durch Tränken des Basismaterials mit einer Lösung oder Suspension eines geeigneten Metallsalzes und Erhöhung des pH auf 10 oder darüber, gegebenenfalls unter Erhitzen, erhalten wird, zur Herstellung eines pharmazeutischen Mittels für die selektive Elimination von anorganischem Phosphat aus Flüssigkeiten, insbesondere aus proteinhaltigen Körperflüssigkeiten wie etwa Vollblut, Plasma, Darminhaltflüssigkeit sowie Dialysierflüssigkeit.

14. Verwendung eines Adsorptionsmaterials wie in einem der vorangehenden Ansprüche definiert, zur Herstellung eines Mittels für die selektive Entfernung von anorganischem Phosphat aus Körperflüssigkeiten, wie etwa Vollblut oder/und Plasma oder zur Herstellung eines Mittels für die Entfernung von anorganischem Phosphat aus Dialyseflüssigkeit in einem extrakorporalen Perfusionssystem.

15. Verwendung nach Anspruch 14,
**dadurch gekennzeichnet,**
daß für die selektive Elimination von anorganischem Phosphat in einem extrakorporalen Perfusionssystem das modifizierte Adsorptionsmaterial ein Basismatarial mit einer Korngröße von 100 bis 500 µm aufweist.

16. Verwendung eines Adsorptionsmaterials wie in einem der Ansprüche 1 bis 11 definiert, zur Herstellung einer oral verabreichbaren Zubereitung für die selektive enterale Elimination von anorganischem Phosphat.

17. Verwendung nach Anspruch 16,
**dadurch gekennzeichnet,**
daß für oral verabreichbare Präparate zur selektiven Elimination von anorganischem Phosphat aus Darminhaltflüssigkeit das Basismaterial für das modifizierte Adsorptionsmaterial eine Korngröße von 5 bis 200 µm aufweist.

18. Verfahren zur Herstellung eines oral zu verabreichenden Arzneimittels für die selektive Entfernung von anorganischem Phosphat,
**dadurch gekennzeichnet,**
daß ein Adsorptionsmaterial wie in einem der Ansprüche 1 bis 11, 13 oder 17 definiert als solches oder in Pulverform verpreßt, mit einer Magensaft-resistenten Schicht überzogen oder in eine säurestabile Kapsel gefüllt wird.

19. Vorrichtung zur selektiven Elimination von anorganischem Phosphat aus wäßrigen Flüssigkeiten, umfassend ein mit einem Zulauf und einem Ablauf versehenes Gehäuse, das eine pharmazeutische Zusammensetzung nach einem der Ansprüche 1 bis 10 oder 12 enthält.

20. Vorrichtung nach Anspruch 19,
**dadurch gekennzeichnet,**
daß das Gehäuse zylindrisch ist, eine Länge von 1 bis 40 cm und einen Durchmesser von 3 bis 20 cm besitzt und an seinen stirnseitigen Enden mit Deckeln versehen ist, die einen Zu- bzw. Ablaufstutzen aufweisen.

21. Vorrichtung nach Anspruch 19 oder 20,
**dadurch gekennzeichnet,**
daß sich am Zu- und Ablauf des Gehäuses Siebe mit einer Porenweite von 10 bis 300 mm befinden.

22. Vorrichtung nach einem der Ansprüche 19 bis 21,
**dadurch gekennzeichnet,**
daß das Gehäuse mittels Strahlen oder Hitze sterilisierbar ist.


**Claims**

1. Composition for use as a pharmaceutical preparation for the selective elimination or inorganic phosphate from liquids,
**wherein**
the composition is insoluble in water and contains an adsorbent material modified with polynuclear metal oxyhydroxides.

2. Composition as claimed in claim 1,
**wherein**
the base material for the modified adsorbent material has organic or/and inorganic hydroxyl groups.

**3.** Composition as claimed in claim 1 or 2,
**wherein**
the base material for the modified adsorbent material has a porous structure with a molecular exclusion limit of $10^2$ to $10^6$ Daltons.

**4.** Composition as claimed in claim 3,
**wherein**
the molecular exclusion limit is less than $10^4$ Daltons.

**5.** Composition as claimed in one of the claims 1 to 4,
**wherein**
the porous base material for the modified adsorbent material has an average particle size between 5 and 500 µm.

**6.** Composition as claimed in one of the claims 1 to 5,
**wherein**
the base material is a natural, semisynthetic or synthetic, linear or/and branch-chained, soluble or insoluble polyhydroxy compound.

**7.** Composition as claimed in one of the claims 1 to 6,
**wherein**
the modified adsorbent material has a porous base material selected from the group comprising materials based on silicates or silicon dioxide in particular glyceryl-modified silica gels, glyceryl-modified glasses (CPG), cross-linked carbohydrates, organic polymers or copolymers.

**8.** Composition as claimed in one of the claims 1 to 7,
**wherein**
the base material is composed of porous silica gels, organic polymers or copolymers which are modified with polysaccharide, in particular dextran.

**9.** Composition as claimed in one of the claims 1 to 8,
**wherein**
the base material is selected from agarose, dextran, dextrin, cellulose or/and polyvinyl alcohol.

**10.** Composition as claimed in one of the previous claims,
**wherein**
the adsorbent material is modified with $Fe^{III}$ oxyhydroxide in a polynuclear form.

**11.** Composition as claimed in one of the previous claims,
**wherein**
it is in a form that can be administered orally.

**12.** Composition as claimed in one of the claims 1 to 10,
**wherein**
it is a component of an extracorporeal perfusion system.

**13.** Use of an adsorbent material as defined in one of the previous claims which is obtained by impregnating the base material with a solution or suspension of a suitable metal salt and increasing the pH to 10 or above optionally while heating, to produce a pharmaceutical preparation for the selective elimination of inorganic phosphate from liquids in particular from body fluids containing protein such as whole blood, plasma, liquid contents of the intestine as well as from dialysis fluid.

**14.** Use of an adsorbent material as defined in one of the previous claims to produce an agent for the selective removal of inorganic phosphate from body fluids such as whole blood or/and plasma or to produce an agent for the removal of inorganic phosphate from dialysis fluids in an extracorporeal perfusion system.

**15.** Use as claimed in claim 14,
**wherein**
the modified adsorbent material has a base material with a particle size of 100 to 500 µm for the selective elimination of inorganic phosphate in an extracorporeal perfusion system.

**16.** Use of an adsorbent material as defined in one of the claims 1 to 11 for the production of a preparation which can be administered orally for the selective enteric elimination of inorganic phosphate.

**17.** Use as claimed in claim 16,
**wherein**
the base material for the modified adsorbent material has a particle size of 5 to 200 μm for preparations which can be administered orally to selectively eliminate inorganic phosphate from the liquid contents of the intestine.

**18.** Process for the production of a pharmaceutical preparation for oral administration for the selective removal of inorganic phosphate,
**wherein**
an adsorbent material as defined in one of the claims 1 to 11, 13 or 17, as such or pressed into a powder form, is coated with a layer resistant to gastric acid or dispensed into an acid-resistant capsule.

**19.** Device for the selective elimination of inorganic phosphate from aqueous liquids comprising a housing provided with an inlet and an outlet which contains a pharmaceutical composition as claimed in one of the claims 1 to 10 or 12.

**20.** Device as claimed in claim 19,
**wherein**
the housing is cylindrical, has a length of 1 to 40 cm and a diameter of 3 to 20 cm and the front ends of which are provided with caps which have an inlet and outlet tube.

**21.** Device as claimed in claim 19 or 20,
**wherein**
sieves with a pore size of 10 to 300 μm are located at the inlet and outlet of the housing.

**22.** Device as claimed in one of the claims 19 to 21,
**wherein**
the housing can be sterilized by means of irradiation or heat.

**Revendications**

**1.** Composition destinée à l'utilisation comme médicament pour l'élimination sélective du phosphate inorganique dans des liquides, la composition étant insoluble dans l'eau et contenant un matériau d'adsorption modifié avec des oxydes-hydroxydes métalliques polynucléaires.

**2.** Composition selon la revendication 1, caractérisée en ce que le matériau de base pour le matériau d'adsorption modifié contient des groupes hydroxyle organiques et/ou inorganiques.

**3.** Composition selon la revendication 1 ou 2, caractérisée en ce que le matériau de base pour le matériau d'adsorption modifié possède une structure poreuse ayant un seuil d'exclusion moléculaire de $10^2$ à $10^6$ daltons.

**4.** Composition selon la revendication 4, caractérisée en ce que le seuil d'exclusion moléculaire est inférieur à $10^4$ daltons.

**5.** Composition selon l'une des revendications 1 à 4, caractérisée en ce que le matériau de base poreux pour le matériau d'adsorption modifié a une grosseur de grains moyenne comprise entre 5 et 500 μm.

**6.** Composition selon l'une des revendications 1 à 5, caractérisée en ce que le matériau de base est un composé polyhydroxylé naturel, semi-synthétique ou synthétique, linéaire et/ou ramifié, soluble ou insoluble.

**7.** Composition selon l'une des revendications 1 à 6, caractérisée en ce que le matériau d'adsorption modifié comprend un matériau de base poreux du groupe constitué par des matériaux à base de silicate ou de dioxyde de silicium, en particulier des gels de silice modifiés par des groupes glycéryle, des verres modifiés par des groupes glycéryle (CPG), des hydrates de carbone réticulés, des polymères ou des copolymères organiques.

**8.** Composition selon l'une des revendications 1 à 7, caractérisée en ce que le matériau de base est constitué d'un gel de silice poreux, d'un polymère ou copolymère organique, modifié avec un polysaccharide, en particulier du dextrane.

**9.** Composition selon l'une des revendications 1 à 8, caractérisée en ce que le matériau de base est choisi parmi l'agarose, le dextrane, la dextrine, la cellulose et/ou un poly(alcool vinylique).

**10.** Composition selon l'une des revendications précédentes, caractérisée en ce que le matériau d'adsorption est modifié avec de l'oxyde-hydroxyde de Fe$^{III}$ sous forme polynucléaire.

**11.** Composition selon l'une des revendications précédentes, caractérisée en ce qu'elle est sous une forme pouvant être administrée par voie orale.

**12.** Composition selon l'une des revendications 1 à 10, caractérisée en ce qu'elle est un constituant d'un système de perfusion extracorporelle.

**13.** Utilisation d'un matériau d'adsorption tel que défini dans l'une des revendications précédentes, obtenu par un procédé selon lequel on imprègne le matériau de base avec une solution ou une suspension d'un sel métallique approprié et on fait monter le pH à au moins 10, éventuellement en chauffant, pour la préparation d'un agent pharmaceutique destiné à l'élimination sélective du phosphate inorganique dans des liquides, en particulier des liquides corporels contenant des protéines comme, par exemple, le sang total, le plasma, le liquide contenu dans l'intestin, ainsi que dans du liquide de dialyse.

**14.** Utilisation d'un matériau d'adsorption tel que défini dans l'une des revendications précédentes pour la préparation d'un agent destiné à l'élimination sélective du phosphate inorganique dans des liquides corporels comme, par exemple, le sang total et/ou le plasma, ou pour la préparation d'un agent destiné à l'élimination du phosphate inorganique dans le liquide de dialyse d'un système de perfusion extracorporel.

**15.** Utilisation selon la revendication 14, caractérisée en ce que, pour l'élimination sélective du phosphate inorganique dans un système de perfusion extracorporelle, le matériau d'adsorption modifié comprend un matériau de base ayant une grosseur de grains de 100 à 500 µm.

**16.** Utilisation d'un matériau d'adsorption tel que défini dans l'une des revendications 1 à 11 pour la préparation d'une composition pouvant être administrée par voie orale, destinée à l'élimination entérique sélective du phosphate inorganique.

**17.** Utilisation selon la revendication 16, caractérisée en ce que, pour des préparations pouvant être administrées par voie orale destinées à l'élimination sélective du phosphate inorganique dans le liquide contenu dans l'intestin, le matériau de base pour le matériau d'adsorption modifié a une grosseur de grains de 5 à 200 µm.

**18.** Procédé de préparation d'un médicament à administrer par voie orale pour l'élimination sélective du phosphate inorganique, caractérisé en ce que l'on comprime un matériau d'adsorption tel que défini dans l'une des revendications 1 à 11, 13 ou 17, tel quel ou sous forme d'une poudre, on l'enrobe d'une couche résistante au suc gastrique ou on l'introduit dans une capsule stable aux acides.

**19.** Dispositif pour l'élimination sélective du phosphate inorganique dans des liquides aqueux, comprenant une enveloppe munie d'un orifice d'entrée et d'un orifice de sortie, qui contient une composition pharmaceutique selon l'une des revendications 1 à 10 ou 12.

**20.** Dispositif selon la revendication 19, caractérisé en ce que l'enveloppe est cylindrique, en ce qu'elle a une longueur de 1 à 40 cm et un diamètre de 3 à 20 cm et en ce qu'elle est munie aux extrémités frontales de couvercles comportant des tubulures d'entrée et de sortie.

**21.** Dispositif selon la revendication 19 ou 20, caractérisé en ce que des tamis ayant une largeur de pores de 10 à 300 µm se trouvent à l'entrée et à la sortie de l'enveloppe.

**22.** Dispositif selon l'une des revendications 19 à 21, caractérisé en ce que l'enveloppe peut être stérilisée à l'aide de rayons ou de chaleur.

Abb. 1   Versuchsaufbau Zirkulationsexperiment mit Humanblut

A Blutreservoir
B Phosphatadsorberkapsel (V=28 ml)

..................................

Blutmonitor HP 50
(B. Braun Melsungen)
 C Pumpe
 D Blasenfänger
 E Manometer

        -•-•- Luer-Lock-
              Verbindung-
              stücke
Schlauchsystem:
Arterielle Leitung
(H.E.L.P.-Systemset
B. Braun Melsungen)
      ᴛ Dreiwegehahn
      zur Blutentnahme

Abbildung 2     Elimination von anorg. Phosphat durch lösliche Metalloxidhydroxid-
Polyol-Komplexe  aus wäßrigen Phosphatlösungen (10mg Phosphor/100ml)

Adsorbens 1 = HS
Adsorbens 2 = PL